# EUROPEAN PATENT SPECIFICATION

(11) **EP 0 647 618 B1**
(45) Date of publication and mention of the grant of the patent: **02.01.1997**
(21) Application number: 94910427.7
(22) Date of filing: 25.03.1994
(51) Int. Cl.: C07C 237/46, A61K 49/04

(54) **NEW NON IONIC IODINE-CONTAINING DIMERS USEFUL AS X-RAY CONTRAST AGENTS, METHOD FOR THE PREPARATION THEREOF, AND GALENICAL COMPOSITIONS CONTAINING THEM**
NICHT-IONISCHE IOD-DIMERE ALS RÖNTGENKONTRASTMITTEL, VERFAHREN ZU IHRER HERSTELLUNG UND GALENISCHE ZUSAMMENSETZUNGEN DIE DIESE ENTHALTEN
NOUVEAUX DIMERES IODES NON-IONIQUES UTILES COMME AGENTS DE CONTRASTE AUX RAYONS X, PROCEDE DE PREPARATION DE CES NOUVEAUX DIMERES, ET COMPOSITIONS GALENIQUES LES CONTENANT

(30) Priority: 01.04.1993 WO PCT/ES93/00022
(43) Date of publication of application: 12.04.1995
(73) Proprietor: CENTRO INVESTIGACION JUSTESA IMAGEN S.A., E-28028 Madrid (ES)
(72) Inventor: ALONSO SILVA, Ignacio, E-28017 Madrid (ES); BÖHLE, Florian, E-28034 Madrid (ES); CARRETERO COLON, José, Maria, E-28030 Madrid (ES); HARTO MARTINEZ, Juán, R., E-28012 Madrid (ES); KRAUSE, Werner, D-13505 Berlin (DE); MARTIN JIMENEZ, José, L., E-28220 Majadahonda (ES); SPECK, Ulrich, D-13465 Berlin (DE)
(74) Representative: Lehmann Novo, Maria Isabel
(86) International application number: ES9400035
(87) International publication number: WO9422811

(56) References cited:
- EP-A- 0 108 638
- EP-A- 0 308 364
- EP-A- 0 317 492
- WO-A-91/12231
- FR-A- 2 272 640
- GB-A- 1 346 795

## Description

The present invention refers to new non-ionic hexaiodinated dimeric compounds, derived from dicarbamoyl triiodoisophthalic acid, which contain in their molecule a 3 - azapentanediamide acid bridge and can be used as X-ray contrast agents, and are of the general formula I 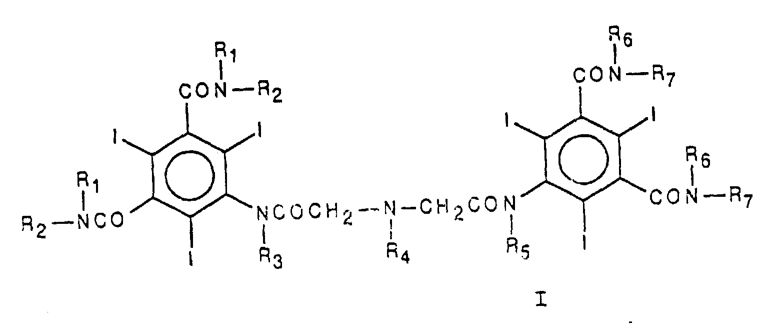 wherein
- R₁ and R₇, whether the same or different, are polyhydroxyalkyl radicals, linear or branched, from C₂ to C₄, with one to three OH groups;
- R₂ and R₆, whether the same or different, are hydrogen, methyl or 2 - hydroxy-ethyl;
- R₃ and R₅, whether the same or different, are hydrogen or methyl;
- R₄ is hydrogen, methyl, methoxymethyl or a polyhydroxyalkyl radical, linear or branched, from C₂ to C₄, with one to three OH groups.

The present invention also includes the possible enantiomers, diastereomers and/or rotomers of the compounds with the general formula I.

The present invention also describes a process for the preparation of these compounds, as well as the galenical compositions containing them, for their use as contrast agents for X-ray diagnostic.

The new compounds of the general formula I are capable of replacing the classic salts of 2,4,6-triiodobenzoic acid as well as the non-ionic monomers as radio - opaque media in radiology, due to their higher level of tolerance and lower incidence of undesirable side-effects. This kind of compounds have practically the same iodine content, but their aqueous solutions show a lower osmotic pressure, which is a known cause of significant disadvantages such as pain and sensation of heat after injection, nausea, migraine, etc. Due to the low tonicity of their solutions, when these compounds are administered via subarachnoid, in myelography and cisternography, they do not cause, in contrast to conventional compounds, arachnoiditis or epileptogenic disorders.

Although the tolerance of the non-ionic monomers is considerably better than that of the salts of 2,4,6-triiodobenzoic acid, aqueous solutions of them at concentrations suitable for diagnosis are still too hypertonic for the blood or cephalorachidian fluid.

This has led to look for new non-ionic bicyclic hexaiodinated compounds (non-ionic dimers), of a reduced osmolality, thus obtaining hypotonic solutions with blood, at concentrations of iodine which are of diagnostic value. Non-ionic iodinated compounds having a dimer structure are described in EP-A-0 108 638, EP-A-0 317 492 and EP-A-0 308 364.

These solutions in general, however, have the disadvantage of being more viscous than those of ionic compounds and non-ionic monomers. Also, many of these products are not sufficiently water-soluble or form supersaturated solutions which crystallize after a while. For this reason, our research has led us to look for new non-ionic bicyclic hexaiodinated compounds which are characterized by a high water solubility, low viscosity and osmolality, as well as high level of intracisternal and intracerebral tolerance. They also show little tendency to produce pain, fever, nausea and vascular endothelium problems.

On the other hand, radiologists need compounds with a wide range of uses: for example, angiography, urography, myelography, cisternography, lymphography, cardiography and gastrography. The products according to this invention fulfil the requirements for the above uses, as they have high water solubility, normally higher than 60% (w/v), which, in some cases, is as high as 100% at 20°C, without showing any tendency to crystallize. They also have a very low general toxicity, stated as the average lethal dose for the rat, DL₅₀.

This information is shown in Table I which shows the values found for some of the preferred compounds according to this invention, namely: ICJ-1192, ICJ-1592 and ICJ-1692, compared with another non-ionic dimer with a different chemical structure, Iodixanol (EP 108.638 Nyeegard and C₀).

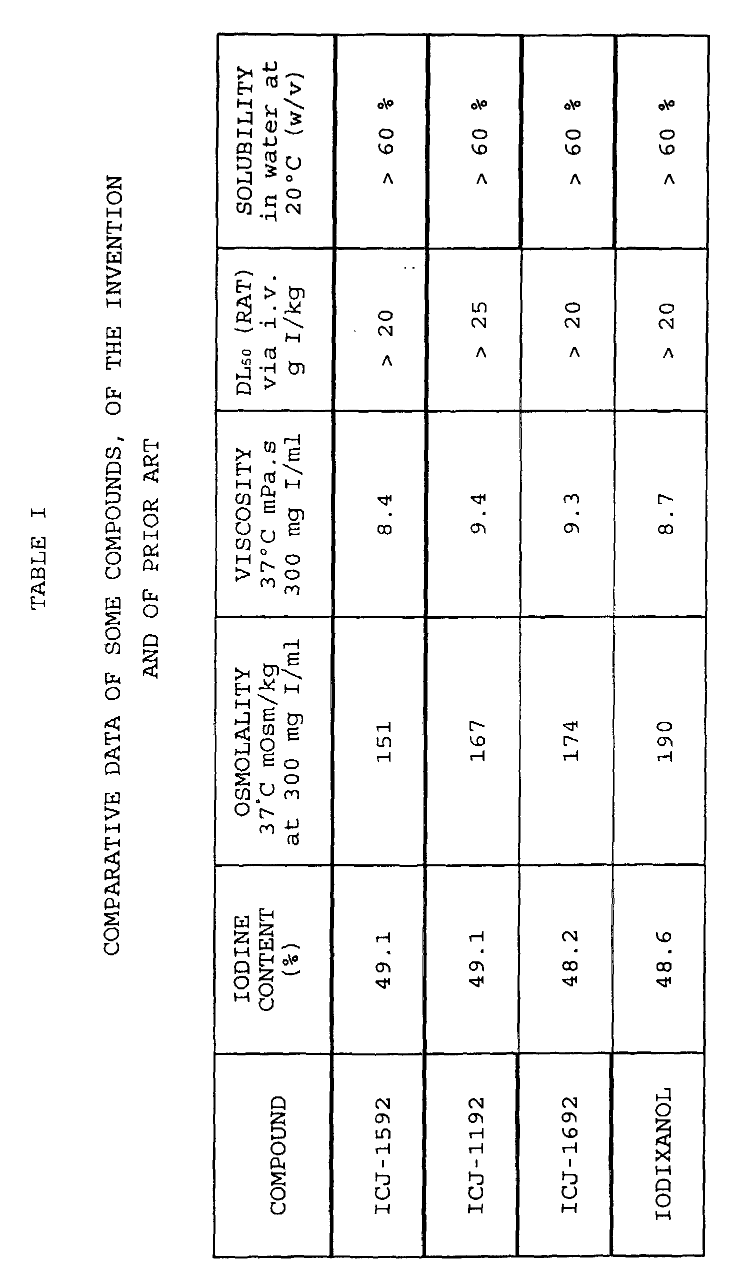

The compounds according to this invention may be used as opacifying media for X-rays in concentrations between 150 and 500 mg I/ml and in doses of 10 to 250 ml, according to the radiological scanning required. These solutions can be prepared along with substances which are acceptable from a pharmaceutical point of view, which are used as stabilizing agents such as sodium and calcium salts of ethylenediaminetetraacetic acid in concentrations of between 0.05 and 2 mM/l, pH-stabilizing agents which are buffers such as trometamol, phosphate, citrate or bicarbonate ions and also balanced solutions of other ions such as C1⁻, Ca²⁺, Mg²⁺, K⁺, Na⁺. In the same way, the hypotonic solutions which are obtained by dissolving the compounds stated in this specification in water can bring about isotonicity by adding an appropiate amount of sodium chloride.

The preparation process of the compounds of the general formula I is a simple one, and is carried out by chemical reaction between a compound of the general formula II 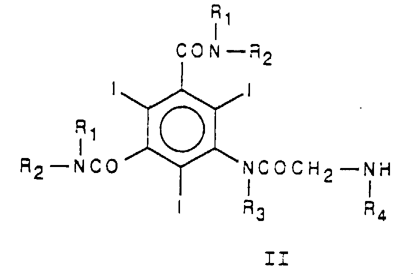 with a halogen derivative of the formula III 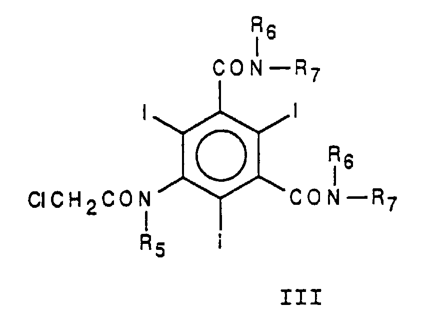 wherein R₁ to R₇ are the same as before in the presence of a basic catalyst.

During the reaction, the OH groups are protected as acetates or dioxepines and, in the case of neighbouring OH groups, as dioxolanes. These protective groups are easily removed by conventional chemical methods such as hydrolysis.

The corresponding compounds of the general formulae II and III are obtained by the methods described in the mentioned examples.

The compounds of the general formula I are easily obtained by means of the procedure indicated. They are purified by treatment with anionic and cationic exchange resins, and by conventional purifying methods, above all crystallization in solvents such as water, methanol etc, and by preparative liquid chromatography.

In the following, it is described the synthesis of the compounds whose chemical structures are displayed along with some of their physico-chemical properties - viscosity, solubility and osmolality, in Table II.

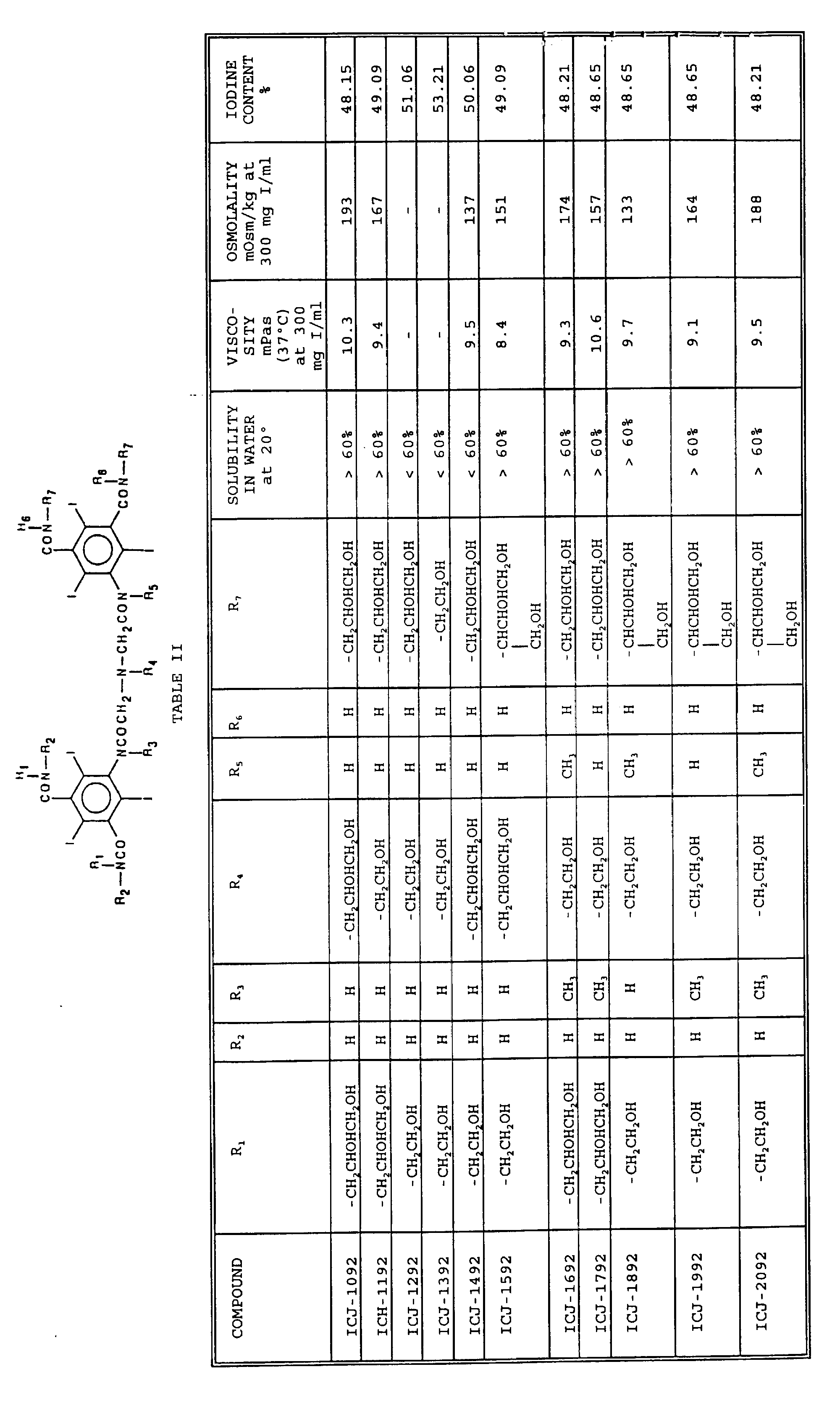

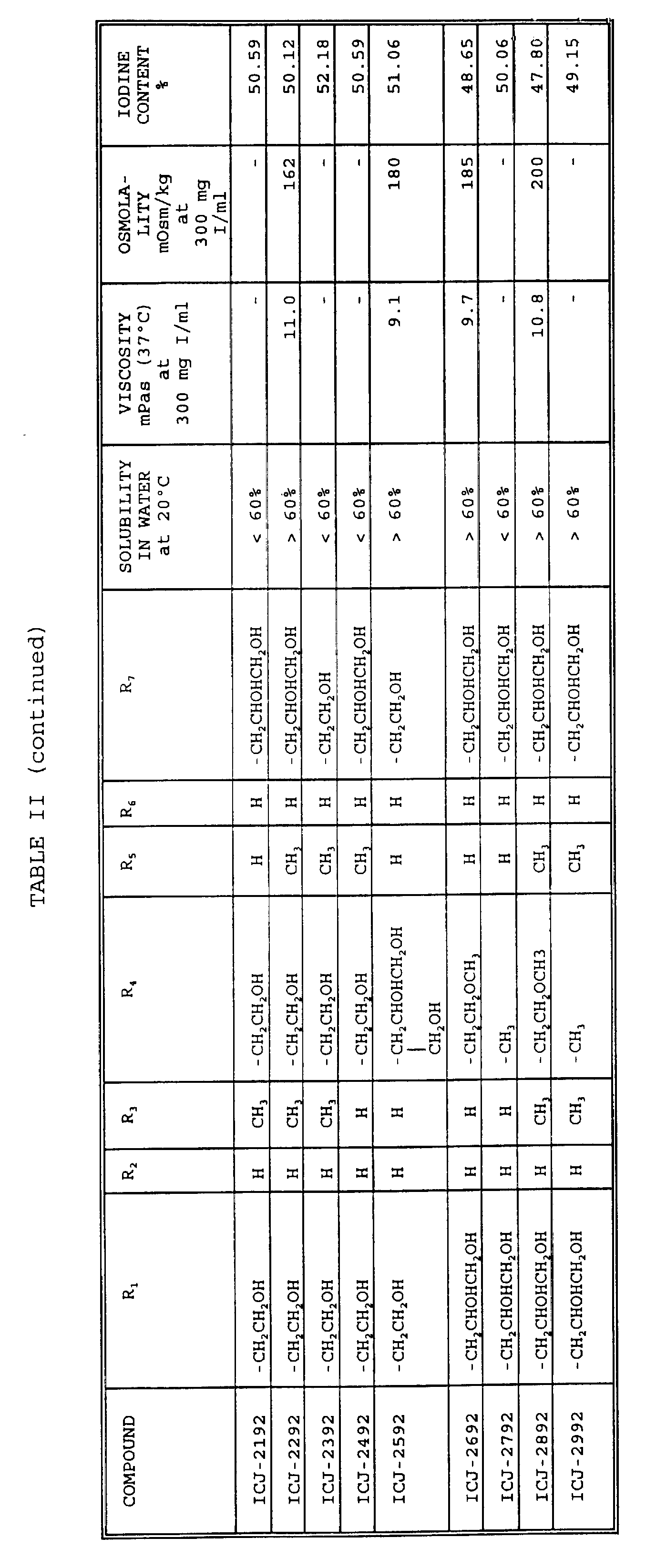

### EXAMPLE 1

### N,N'-Bis-{3,5-bis-[N-(2,3-dihydroxypropyl)carbamoyl]-2,4,6-triiodophenyl}-3-(2,3-dihydroxypropyl)-3-azapentanediamide (ICJ-1092)

### A) 2,2-Dimethyl-5-aminomethyl-1,3-dioxolane hydrochloride (ICJ-115)

182.2g (2 moles) of 3-amino-1,2-propandiol are dissolved in 540 ml of water. Then 35% hydrochloric acid is added until pH 1 - 2 is reached, and the mixture is stirred at the ambient temperature for 15 minutes. The solvent is then evaporated upto dryness. A suspension of the resultant residue is made in 1.2 1 of 2,2 - dimethoxypropane. 15.0 g of p-toluene-sulphonic acid are added and the mixture is vigorously stirred under reflux for half an hour. The precipitated solid is filtered, washed with acetone and dried in a vacuum. 313 g are obtained (93%)

### B) N,N'-Bis-[2,3-(isopropylidenedioxy)propyl]-5-chloroacetamido-2,4,6-triiodoisophthalamide (ICJ-145)

To a solution of 134.4 g (0.2 mole) of 5-chloroacetamido-2,4,6-isophthaloyl chloride in 400 ml of acetone 73.7 g (0.44 mole) of the compound from the example 1A) and 125.9 g (0.44 mole) of sodium carbonate decahydrate are added. The mixture is then heated under reflux for 4 hours. The precipitated solid is filtered and then treated with water at a temperature of 60°C for 2 hours, after which it is treated with acetone at the ambient temperature for an hour. It is then dried in a vacuum and 160 g (93%) are obtained.

### C) N,N'-Bis-[2,3-(isopropylidenedioxy)propyl]-5-{N-[2,3-(isopropylidenedioxy)propyl] aminoacetamido}-2,4,6-triiodoisophthalamide (ICJ-141)

To a suspension of 43 g (0.05 mole) of the compound from the example 1B) in 400 ml of dioxane, 8.3 g (0.05 mole) of potassium iodide, 25.1 g (0.15 mole) of the compound from the example 1A) and 42.9 g (0.15 mole) of sodium carbonate decahydrate are added. The mixture is heated at a temperature of 65 - 70°C for 24 hours. The insoluble salts are filtered out and the solvent is evaporated to dryness. The residue is dissolved in chloroform and is washed with water, 5% sodium bicarbonate and a saturated solution of sodium chloride. It is dried and the solvent evaporated at reduced pressure. 44.3 g (92.6%) are obtained.

### D) N,N'-Bis-{3,5-bis-[N-(2,3-dihydroxypropyl)carbamoyl]-2,4,6-triiodophenyl}-3-(2,3-dihydroxypropyl) -3-azapentanediamide (ICJ-1092)

60.3 g (0.07 mole) of the compound from the example 1B) and 66.9 g (0.07 mole) of the compound 1C) are suspended in 380 ml of dioxane. 11.6 g (0.07 mole) of potassium iodide and 40 g (0.014 mole) of sodium carbonate decahydrate are added to the suspension, which is heated at a temperature of 70 - 75°C for 48 hours. The insoluble salts are filtered out and the residue is dissolved in 400 ml of ethyl acetate and allowed to settle. The crystallized product is filtered out and dried in a vacuum. This product (86 g) is suspended in a methanol - water mixture, acidulated and heated at a temperature of 40 - 45°C until it has been totally dissolved. It is then neutralized and evaporated to dryness. The residue is purified by reverse phase preparative liquid chromatography (PLC) using mixtures water/methanol as an eluant.
80 g (72%) are obtained.

### EXAMPLE 2

### N,N'-Bis-{3,5-bis-[N-(2,3-dihydroxypropyl)carbamoyl]-2,4,6-triiodophenyl}-3-(2-hydroxyethyl)-3-azapentanediamide (ICJ-1192)

### A) N,N'-Bis-[2,3-(isopropylidenedioxy)propyl]-5-[N-(2-hydroxyethyl)aminoacetamido]-2,4,6-triiodoisophthalamide (ICJ-165)

To a suspension of 215.4 g (0.250 mole) of the compound described in example 1B in 650 ml of dioxane, 46 g (0.753 mole) of 2 - aminoethanol, 72 g (0.251 mole) of sodium carbonate decahydrate and 45 g (0.250 mole) of potassium iodide are added. The reaction mixture is heated under reflux for 3 hours. We ensure that the reaction is complete by using thin-layer chromatography (TLC) employing a chloroform-methanol (5:1) mixture as an eluant. The resultant product is cooled, filtered and the filtrate evaporated to dryness. The residue is dissolved in water, extracted with chloroform and the chloroform extract evaporated to dryness. Thus, 204 g (yield = 92%) of the desired product are obtained.

### B) N,N'-Bis-{3,5-bis-[N-(2,3-dihydroxypropyl)carbamoyl]-2,4,6-triiodophenyl}-3-(2-hydroxyethyl)-3-azapentanediamide (ICJ-1192)

A mixture of 17.7 g (0.020 mole) of the compound described in part 2A, 17.2 g (0.020 mole) of the chlorine-derivative of example 1B, 5.8 g (0.020 mole) of sodium carbonate decahydrate and 3.4 g (0.020 mole) of potassium iodide in 95 ml of dioxane is heated under reflux for 24 hours. It is allowed to cool, the salts filtered out and the filtrate evaporated to dryness at reduced pressure. The residue is dissolved in 60 ml of methanol and 60 ml of water and hydrolyzed by means of concentrated hydrochloric acid up to a pH of 1 at a temperature of 50°C. It is neutralized by a solution of sodium hydroxide, filtered and the filtrate concentrated to dryness at reduced pressure. The resultant product is purified by reverse phase preparative liquid chromatography using mixtures of methanol and water as an eluant.
Yield = 17 g (55%)

### EXAMPLE 3

### N-{3,5-Bis-[N-(2,3-dihydroxypropyl)carbamoyl]-2,4,6-triiodophenyl}-N'-{3,5-bis- [N-(2-hydroxyethyl)carbamoyl]-2,4,6-triiodophenyl}-3-(2-hydroxyethyl)-3-azapentanediamide (ICJ-1292)

To a solution of 100 g (0.13 mole) of N,N'-bis-(2-hydroxyethyl)-5-(2-hydroxyethylaminoacetamide)-2,4,6-triiodoisophthalamide (described in DOS 2928417) in 580 ml of dimethylformamide, 112 g (0.13 mole) of the compound from the example 1B), 21.6 g (0.13 mole) of potassium iodide and 74.4 g (0.26 mole) of sodium carbonate decahydrate are added. This is heated at a temperature of 70 - 75°C for 48 hours. The insoluble salts are filtered out and the solvent is evaporated to dryness. The resultant residue is triturated with chloroform (800 ml), filtered and dried in a vacuum. The obtained product (190 g) is dissolved in 500 ml of water and 500 ml of methanol and hydrolyzed in an acid medium (pH 1 - 2) at 40°C. Once neutralized it is evaporated to dryness and the residue purified by reverse phase preparative liquid chromatography using mixtures of methanol and water as an eluant.
72 g (39%) are obtained.

### EXAMPLE 4

### N,N'-Bis-{3,5-bis-[N-(2-hydroxyethyl)carbamoyl]-2,4,6-triiodophenyl}-3-(2-hydroxyethyl)-3-azapentanediamide (ICJ-1392)

### A) N,N'-Bis-(2-hydroxyethyl)-5-chloroacetamide-2,4,6-triiodoisophthalamide (ICJ-167)

A mixture of 268.9 g (0.40 mole) of 5-chloroacetamido-2,4,6-triiodoisophthaloyl-chloride, 51.6 g (0.84 mole) of 2-aminoethanol and 240.4 g (0.84 mole) of sodium carbonate decahydrate in 1.1 1 of acetone is heated under reflux with vigorous stirring for 3 hours. It is allowed to cool, filtered and the resultant solid treated with 750 ml of water at 70°C for 1 hour. It is filtered, washed with acetone and the product obtained dried in a vacuum at 50°C over P₂ O₅.
Yield = 274 g (95%)

### B) N,N'-Bis-{3,5-bis-[N-(2-hydroxyethyl)carbamoyl]-2,4,6-triiodophenyl}-3-(2-hydroxyethyl)-3-azapentanediamide (ICJ-1392)

A mixture of 100 g (0.134 mole) of N,N'-bis-(2-hydroxyethyl)-5-(2-hydroxyethylaminoacetamide)-2,4,6-triiodoisophthalamide, 96.7 g (0.134 mole) of the chlorine derivative described in the previous section (example 4A), 38.4 g (0.134 mole) of sodium carbonate decahydrate and 22.3 g (0.134 mole) of potassium iodide in 450 ml of dimethylformamide is heated at 70°C with vigorous stirring for 24 hours. It is allowed to cool, the salts are filtered out and the filtrate evaporated to dryness at reduced pressure. The product obtained is purified by reverse phase prepartive liquid chromatography using mixtures of methanol and water as an eluant.
Yield = 134.1 g (70%)

### EXAMPLE 5

### N-{3,5-bis-[N-(2,3-dihydroxypropyl)carbamoyl]-2,4,6-triiodophenyl}-N'-{3,5-bis-[N-(2-hydroxyethyl)carbamoyl]-2,4,6-triio dophenyl}-3-(2,3-dihydroxypropyl)-3-azapentanediamide (ICJ 1492)

A mixture of 124.3 g (0.13 mole) of the compound described in example lC, 93.8 g (0.13 mole) of the chlorine derivative of example 4A, 74.4 g (0.26 mole) of sodium carbonate decahydrate and 21.6 g (0.13 mole) of potassium iodide in 450 ml of dimethylformamide (DMF) is heated to 70°C with vigorous stirring for a period of 2 days. It is allowed to cool, the salts are filtered out and the filtrate evaporated to dryness at reduced pressure. The residue is triturated with 1 litre of chloroform for two hours, filtered and dried in a vacuum, the yield being 212 g (99.4%) of N-{3,5-bis-[N-(2,3-(isopropylidenedioxy)propyl)carbamoyl]-2,4,6-triiodophenyl}-N'-{3,5-bis-[N-(2-hydroxyethyl)carbamoyl]-2,4,6-triiodophenyl}-3-[2,3-(isopropylidenedioxy)propyl]-3-azapentanediamide.

This product is then dissolved in 500 ml of methanol and 500 ml of water and hydrolyzed using concentrated hydrochloric acid up to pH 1 at 50°C for 3 hours. It is neutralized using a solution of sodium hydroxide (up to pH 6.5) and decolourized using 25 g of active charcoal for 2 hours. It is filtered and the filtrate concentrated to dryness at reduced pressure. The resultant product is purified by reverse phase preparative liquid chromatography (PLC), using mixtures of methanol and water as an eluant.
Yield = 115 g (58%)

### EXAMPLE 6

### N-{3,5-Bis-[N-(1-hydroxymethyl-2,3-dihydroxypropyl)carbamoyl]-2,4,6-triiodophenyl}-N'-{3,5-bis-[N-(2-hydroxyethyl)car bamoyl]-2,4,6-triiodophenyl}-3-(2-hydroxyethyl)-3-azapentane diamide (ICJ 1592)

### A) N,N'-Bis-(1-hydroxymethyl-2,3-dihydroxypropyl)-5-chloroacetamide-2,4,6-triiodoisophthalamide (ICJ-130)

134.4 g (0.2 mole) of 5-chloroacetylamino-2,4,6-triiodoisophthaloyl chloride are dissolved in 670 ml of acetone. Then 80.6 g (0.5 mole) of 5-amino-2,2-dimethyl-5-hydroxy-1,2-dioxepine and 143 g (0.5 mole) of sodium carbonate decahydrate are added. This mixture is heated under reflux for 5 hours. The insoluble salts are filtered out and the solvent evaporated to dryness. The residue is suspended in 300 ml of water and is acidulated (pH=1-2) with concentrated hydrochloric acid. It is stirred for an hour at the ambient temperature and neutralized. It is then evaporated to dryness and the residue purified by means of precipitation using a methanol-isopropanol mixture.
Yield = 150 g (89.1%).

### B)N-{3,5-Bis-[N-(1-hydroxymethyl-2,3-dihydroxypropyl)carbamoyl]-2,4,6-triiodophenyl}-N'-{3,5-bis-[N-(2-hydroxy ethyl)carbamoyl]-2,4,6-triiodophenyl}-3-(2-hydroxyethyl)-3 -azapentanediamide (ICJ-1592)

91.7 g (0.123 moles) of N,N'-bis-(2-hydroxyethyl)-5-(2-hydroxyethylaminoacetamido)-2,4,6-triiodoisophthalamide are dissolved in 640 ml of DMF. Then 103.5 g (0.123 mole) of the compound from the example 6A), 20.4 g (0.123 mole) of potassium iodide and 70.4 g (0.246 mole) of sodium carbonate decahydrate are added. The mixture is heated at 70 - 75 °C for 24 hours. The insoluble salts are filtered out and the filtrate evaporated to dryness. The residue is dissolved in methanol and the product precipitated out using isopropanol, after that it is purified by reverse phase PLC with water-methanol mixtures as an eluant.
79 g (41.4%) are obtained.

### EXAMPLE 7

### N,N'-Bis-{3,5-bis-[N-(2,3-dihydroxypropyl)carbamoyl]-2,4,6-triiodophenyl}-N,N'-dimethyl-3-(2-hydroxethyl)-3-azapentane diamide (ICJ-1692)

### A) N,N'-Bis-[2,3-(isopropylidenedioxy)propyl]-5-chloroacetyl(methyl)amino-2,4,6-triiodoisophthalamide (ICJ-183)

To a suspension of 68.6 g (0.100 mole) of 5-chloroacetyl(methyl)amino-2,4,6-triiodoisophthaloyl chloride in 200 ml of acetone, 41.9 g (0.250 mole) of the compound from the example 1A and 71.5 g (0.250 mole) of sodium carbonate decahydrate are added. The reaction mixture is heated under reflux for 6 hours, making sure that the reaction is complete by employing TLC and a chloroform-methanol mixture (10:1) as an eluant. The mixture is allowed to cool, filtered, and the solid washed with water and acetone and dried in a vacuum. The product obtained is purified by the recrystallization in ethyl acetate.
Yield = 75 g (85%)

### B)N,N'-Bis-[2,3-(isopropylidenedioxy)propyl]-5-[(2-hydroxyethyl)aminoacetyl(methyl)amino]-2,4,6-triiodoisophthalamide (ICJ-184)

To a suspension of 41.7 g (0.048 mole) of the compound described in the previous section (example 7a) in 130 ml of dioxane 8.8 g (0 144 mole) of 2-aminoethanol, 13.7 g (0.048 mole) of sodium carbonate decahydrate and 7.9 g (0.048 mole) of potassium iodide are added. The reaction mixture is heated under reflux for 3 hours, making sure that the reaction is complete by thin-layer chromatography (TLC), using a chloroform-methanol mixture (5:1). It is allowed to cool, filtered and the filtrate evaporated to dryness at reduced pressure. The residue is dissolved in water (120 ml), extracted with chloroform (3 x 200 ml) and the chloroform extract evaporated to dryness. Thus are obtained 38.6 g (yield = 90%) of the title product.

### C) N,N'-Bis-{3,5-bis-[N-(2,3-dihydroxypropyl)carbamoyl]-2,4,6-triiodophenyl}-N,N'-dimethyl-3-(2-hydroxyethyl)-3 -azapentanediamide (ICJ-1692)

A mixture of 45.1 g (0.050 mole) of the compound described in the previous paragraph (example 7B), 43.8 g (0.050 mole) of the chlorine derivative in example 7A, 14.3 g (0.050 mole) of sodium carbonate decahydrate and 8.3 g (0.050 mole) of potassium iodide in 140 ml of dioxane is heated under reflux for 24 hours. It is allowed to cool, the salts filtered out and the filtrate evaporated to dryness at reduced pressure. The residue is dissolved in 150 ml of methanol and 150 ml of water and hydrolyzed using concentrated hydrochloric acid up to pH = 1 at 50°C. It is neutralized using sodium hydroxide solution, filtered and the filtrate concentrated to dryness at reduced pressure. The resultant product is purified by reverse phase PLC using mixtures of methanol and water as an eluant.
Yield = 40 g (51%)

### EXAMPLE 8

### N,N'-Bis-{3,5-bis-[N-(2,3-dihydroxypropyl)carbamoyl]-2,4,6-triiodophenyl}-N-methyl-3-(2-hydroxyethyl)-3-azapentanediami de (ICJ 1792)

A mixture of 30.4 g (0.034 mole) of the compound described in example 1C, 30.0 g (0.034 mole) of the chlorine derivative of example 7A, 9.9 g (0.035 mole) of sodium carbonate decahydrate and 5.7 g (0.034 mole) of potassium iodide in 95 ml of dioxane is heated under reflux for 24 hours. It is allowed to cool, the salts filtered out and the filtrate evaporated to dryness at reduced pressure. The residue is dissolved in 180 ml of methanol and 180 ml of water and hydrolyzed using concentrated hydrochloric acid up to pH = 1 at 50°C. It is neutralized using sodium hydroxide solution, filtered and the filtrate concentrated to dryness at reduced pressure. The resultant product is purified by reverse phase PLC using mixtures of methanol and water as an eluant.
Yield = 22.0 g (46%)

### EXAMPLE 9

### N{3,5-Bis-[N-(1-hydroxymethyl-2,3-dihydroxypropyl)carbamoyl]-2,4,6-triiodophenyl}-N-methyl-N'-{3,5-bis-[N-(2-hydroxy ethyl)carbamoyl]-2,4,6-triiodophenyl}-3-(2-hydroxyethyl)-3 -azapentanediamide (ICJ 1892)

A mixture of 100 g (0.134 mole) of N,N'-bis-(2-hydroxyethyl)-5-(2-hydroxyethylaminoacetamido)-2,4,6-triiodoisophthalamide, 125.4 g (0.134 mole) of N,N'-bis-(7-hydroxy-4,4-dimethyl-3,5-dioxepinyl)-5-[chloroacetyl(methyl)amino]-2,4,6-triiodoisophthalamide, 76.7 g (0.268 mole) of sodium carbonate decahydrate and 22.3 g (0.134 mole) of potassium iodide in 450 ml of DMF is heated at 70°C, with vigorous stirring, for 2 days. It is allowed to cool, the salts filtered out and the filtrate evaporated to dryness at reduced pressure. The residue is triturated with 1 litre of chloroform for 2 hours, filtered and dried in a vacuum, being obtained 219.4 g (99.5%) of N-{3,5-bis-[N-(7-hydroxy-4,4-dimethyl-3,5-dioxyepinyl)carbamoyl]-2,4,6-triiodophenyl}-N'-{3,5-bis-[N-(2-hydroxyethyl)carbamoyl]-2,4,6-triiodophenyl}-3-(2-hydroxyethyl)-3-azapentanediamide.

This product is then dissolved in 500 ml of methanol and 500 ml of water and hydrolyzed using concentrated hydrochloric acid up to pH = 1 at 50°C for 3 hours. It is neutralized with 50% sodium hydroxide solution (up to pH 6.5) and decolourized by means of activated charcoal for a period of 2 hours. It is filtered and the filtrate concentrated to dryness at reduced pressure. The resultant product is purified by reverse phase PLC, employing methanol-water mixtures as an eluant.
Yield = 84.6 g (40%)

### EXAMPLE 10

### N-{3,5-Bis-[N-(1-hydroxymethyl-2,3-dihydroxypropyl)carbamoyl]-2,4,6-triiodophenyl}-N'-methyl-N'-{3,5-bis-[N-(2-hy droxyethyl)carbamoyl]-2,4,6-triiodophenyl}-3-(2-hydroxy ethyl)-3-azapentanediamide (ICJ 1992)

### A) N,N'-Bis-(2-hydroxyethyl)-5-[(2-hydroxyethyl)aminoacetyl(methyl) amino]-2,4,6-triiodoisophthalamide (ICJ-195)

A mixture of 137.2 g (0.20 mole) of 5-chloroacetyl (methyl)amino-2,4,6-triiodoisophthaloyl chloride, 61.1 g (1 mole) of 2-aminoethanol and 114.5 g (0.40 mole) of sodium carbonate decahydrate in 550 ml of DMF is heated to 50 - 60°C, with vigorous stirring, for 2 hours. Then 33.2 g of potassium iodide are added and the heating of the mixture continues until a total of 24 hours has elapsed. It is allowed to cool, the salts filtered out and the filtrate evaporated to dryness at reduced pressure. The residue obtained is triturated with 700 ml of isopropanol, it is filtered out and the resulting solid is submitted to successive treatments with ethanol and isopropanol until the excess of 2-aminoethanol disappears. It is then dried in a vacuum.
Yield = 91.2 g (60%)

### B)N-{3,5-Bis-[N-(1-hydroxymethyl-2,3-dihydroxypropyl)carbamoyl]-2,4,6-triiodophenyl}-N'-methyl-N'-{3,5-bis-[N-(2-hydro xyethyl)carbamoyl]-2,4,6-triiodophenyl}-3-(2-hydroxyethyl)-3 -azapentanediamide (ICJ-1992)

To a solution of 48.0 g (0.063 mole) of the compound from the example 10A) and 53.0 g (0.063 mole) of the compound from the example 6A) in 340 ml of DMF, 10.4 g (0.063 mole) of potassium iodide and 36.0 g (0.126 mole) of sodium carbonate decahydrate are added. This mixture is then heated at 70 - 75°C for 2 days. The insoluble salts are filtered out and the filtrate evaporated to dryness. The residue is dissolved in methanol and precipitated out using isopropanol. The solid obtained - a quantity of 67 g (68%) - is purified by reverse phase PLC using methanol-water mixtures as an eluant. 41 g (41%) are obtained.

### EXAMPLE 11

### N-{3,5-Bis-[N-(1-hydroxymethyl-2,3-dihydroxypropyl)carbamoyl]-2,4,6-triiodophenyl}-N'-{3,5-bis-[N-(2-hydroxy ethyl)carbamoyl]-2,4,6-triiodophenyl}-N,N'-dimethyl-3-(2 -hydroxyethyl)-3-azapentanediamide (ICJ 2092)

A mixture of 55.5 g (0.073 mole) of the compound from the example 10A), 58.1 g (0.073 mole) of N,N'-bis-(7-hydroxy-4,4-dimethyl-3,5-dioxepinyl)-5-[chloroacetyl(methyl)amino]-2,4,6-triiodoisophthalamide, 41.8 g (0.14 mole) of sodium carbonate decahydrate and 12.1 g (0.073 mole) of potassium iodide in 340 ml of dimethylformamide is heated to 70-75°C for 2 days. The insoluble salts are filtered out and the filtrate evaporated to dryness at reduced pressure. The residue is dissolved in a methanol/water mixture and is hydrolyzed by means of 35% hydrochloric acid at pH 1-2 at 50°C for 1 hour. It is then neutralized (pH 6.5) and evaporated to dryness. The residue is precipitated out using a methanol/isopropanol mixture, thus obtaining 106 g (96%) of a solid which is purified by reverse phase PLC using methanol-water mixtures as an eluant. 57 g (49%) are obtained.

### EXAMPLE 12

### N-{3,5-Bis-[N-(2,3-dihydroxypropyl)carbamoyl]-2,4,6-triiodophenyl}-N'-methyl-N'-{3,5-bis-[N-(2-hydroxyethyl)carbamoyl]-2,4,6-triiodophenyl}-3-(2-hydroxyethyl)-3-azapentanediamide (ICJ 2192)

53.2 g (0.07 mole) of the compound from the example 10A) and 60 g (0.07 mole) of the compound from the example 1B) are dissolved in 380 ml of dimethylformamide. Then 11.6 g (0.07 mole) of potassium iodide, 9.2 ml (0.07 mole) of triethylamine and 12.6 ml (0.07 mole) of water are added. This is heated at 70 - 75°C for 2 days and the solvent evaporated at reduced pressure. The residue is dissolved in a methanol/water mixture and hydrolyzed at pH 1-2 using 35% hydrochloric acid at 40°C for 1 hour. It is neutralized and the resultant solution evaporated to dryness. The precipitation using a methanol/isopropanol mixture gives 101 g (96%) of product which is purified by reverse phase PLC using methanol/water mixtures as an eluant. 50.1 g (47%) are obtained.

### EXAMPLE 13

### N-{3,5-Bis-[N-(2,3-dihydroxypropyl)carbamoyl]-2,4,6-triiodophenyl}-N'-{3,5-bis-[N-(2-hydroxyethyl)carbamoyl]-2,4,6-triio dophenyl}-N,N'-dimethyl-3-(2-hydroxyethyl)-3-azapentanediami de (ICJ 2292)

### A) N,N'-Bis-(2-hydroxyethyl)-5-chloroacetyl(methyl)amino-2,4,6-triiodoisophthalamide (ICJ-194)

A suspension of 137.2 g (0.20 mole) of 5-chloroacetyl (methyl)amino-2,4,6-triiodoisophthaloyl chloride, 25.0 g (0.41 mole) of 2-aminoethanol and 117.3 g (0.41 mole) of sodium carbonate decahydrate in 550 ml of acetone is heated under reflux for 3-4 hours with vigorous stirring. The salts are filtered out and the filtrate evaporated to dryness at reduced pressure. The residue obtained is triturated with 700 ml of water at 40-50°C for an hour, allowed to cool, filtered and washed with water. The resultant solid is dried in a vacuum at 50°C.
Yield = 120 g (82%)

### B) N-{3,5-Bis-[N-(2,3-dihydroxypropyl)carbamoyl]-2,4,6-triiodophenyl}-N'-{3,5-bis-[N-(2-hydroxyethyl)carbamoyl]-2,4,6 -triiodophenyl}-N,N'-dimethyl-3-(2-hydroxyethyl)-3-azapentane diamide (ICJ-2292)

To a solution of 40 g (0.044 mole) of the compound described in the example 7B, 6.2 ml (4.5 g; 0.044 mole) of triethylamine and 8 ml of water in 220 ml of DMF, 32.7 g (0.044 mole) of the chlorine derivative described in the previous paragraph 13A and 7.4 g (0.044 mole) of potassium iodide are added with energic stirring. The reaction mixture is heated at 70-75°C for two days. It is then allowed to cool, the salts filtered out and the filtrate evaporated to dryness at reduced pressure and then dried in a vacuum. 71 g (quantitative yield) of N-{3,5-bis-[N-(2,3-(isopropylidenedioxy)propyl)carbamoyl]-2,4,6-triiodophenyl}-N'-{3,5-bis-[N-(2-hydroxyethyl)carbamoyl]-2,4,6-triiodophenyl}-N,N'-dimethyl-3-(2-hydroxyethyl)-3-azapentanediamide are obtained. This is then converted into the desired product by hydrolysis with concentrated hydrochloric acid to pH 1 in 300 ml of water at 50°C for 2 hours. It is then neutralized using sodium hydroxide solution to pH 6.5 and concentrated to dryness at reduced pressure. The resultant product is purified by reverse phase PLC using methanol-water mixtures as an eluant.
Yield = 35.1 g (52%)

### EXAMPLE 14

### N,N'-Bis-{3,5-bis-[N-(2-hydroxyethyl)carbamoyl]-2,4,6-triiodophenyl}-N,N'-dimethyl-3-(2-hydroxyethyl)-3-azapentanediami de (ICJ 2392)

A mixture of 50 g (0.066 mole) of the amino derivative described in the example 10A, 48.4 g (0.066 mole) of the chlorine derivative disclosed in the example 13A, 20.7 g (0.072 mole) of sodium carbonate decahydrate and 10.9 g (0.066 mole) of potassium iodide in 300 ml of DMF is heated at 70-75°c with vigorous stirring for 24 hours. It is allowed to cool, the salts filtered out and the filtrate evaporated to dryness at reduced pressure. The product obtained is purified by reverse phase PLC using methanol/water mixtures as an eluant.
Yield = 67.3 g (70%)

### EXAMPLE 15

### N-{3,5-Bis-[N-(2,3-dihydroxypropyl)carbamoyl]-2,4,6-triiodophenyl}-N-methyl-N'-{3,5-bis-[N-(2-hydroxyethyl)carbamoyl] -2,4,6-triiodophenyl}-3-(2-hydroxyethyl)-3-azapentanediamide (ICJ 2492)

67.9 g (0.091 mole) of N,N'-bis-(2-hydroxyethyl)-5-(2-hydroxyethylaminoacetamido)-2,4,6-triiodoisophthalamide and 80 g (0.091 mole) of the compound from the example 7A) are dissolved in 500 ml of dimethylformamide. Then 15.1 g (0.091 mole) of potassium iodide, 12.8 ml (0.091 mole) of triethylamine and 16.4 ml (0.91 mole) of water are added to the mixture. This is then heated at 70 - 75°C for 2 days and the solvent evaporated off at reduced pressure. The residue is dissolved in a water/methanol mixture and hydrolyzed at pH 1-2 using 35% hydrochloric acid at 40°C for 2 hours. It is neutralized and the resultant solution evaporated to dryness. A methanol-isopropanol mixture is then used to precipitate out the residue, being obtained 125 g (91%) of the product, which is then purified by means of reverse phase PLC using methanol/water mixtures as an eluant.
Yield = 65 g (47%)

### EXAMPLE 16

### N,N'-Bis-{3,5-bis-[N-(2-hydroxyethyl)carbamoyl]-2,4,6-triiodophenyl}-3-(1-hydroxymethyl-2,3-dihydroxypropyl)-3-azapenta nediamide (ICJ 2592)

75.0 g (0.104 mole) of the chlorine derivative described in example 4A and 8.1 g (0.050 mole) of 5-amino-2,2-dimethyl-4-hydroxy-1,3-dioxepine are dissolved in 225 ml of dimethylformamide. 17.3 g (0.104 mole) of potassium iodide, 14.5 ml (0.104 mole) of triethylamine and 10.8 ml of water are then added. The mixture is heated at 70-75°C for 2 days and the solvent evaporated off at reduced pressure. The residue is dissolved in a water/methanol mixture and hydrolyzed using 35% hydrochloric acid at pH 1-2 at 40°C for 2 hours. It is then neutralized and the resultant solution evaporated to dryness. The residue is purified by reverse phase PLC using water-methanol mixtures as an eluant.
Yield = 32.8 g (44%)

### EXAMPLE 17

### N,N'-Bis-{3,5-bis-[N-(2,3-dihydroxypropyl)carbamoyl]-2,4,6-triiodophenyl}-3-(2-methoxyethyl)-3-azapentanediamide (ICJ 2692)

To a suspension of 172.3 g (0.2 mole) of the chlorine derivative described in example 1B and 7.5 g (0.1 mole) of 2-methoxyethylamine in 520 ml of dioxane, 33.2 g (0.2 mole) of potassium iodide and 20.3 g (0.2 mole) of triethylamine are added. The reaction mixture is then heated at 80 - 85°C for 16 hours, filtered and the solvent evaporated off at reduced pressure. The residue is dissolved in a water/methanol mixture and the protecting groups are hydrolyzed using hydrochloric acid at pH 1-2 and 40°C for 3 hours. It is neutralized and the resultant solution evaporated to dryness. The residue is purified by reverse phase liquid chromatography using methanol-water mixtures as an eluant.
Yield = 82.0 g (52%)

### EXAMPLE 18

### N,N'-Bis-{3,5-bis-[N-(2,3-dihydroxypropyl)carbamoyl]-2,4,6-triiodophenyl}-3-methyl-3-azapentanediamide (ICJ 2792)

To a suspension of 172.3 g (0.2 mole) of the chlorine derivative described in example 1B and 3.1 g (0.1 mole) of methylamine (7.75 g of a 40 weight % aqueous solution)in 520 ml of dioxane, 33.2 g (0.2 mole) of potassium iodide and 20.3 g (0.2 moles) of triethylamine are added. The reaction mixture is then heated at a temperature of 80-85°C for 24 hours, filtered and then the solvent evaporated off at reduced pressure. The residue is dissolved in a water/methanol mixture and the protecting groups hydrolyzed with hydrochloric acid at pH 1-2 and 40°C for 3 hours. It is neutralized and the resultant solution evaporated to dryness. The residue is purified by reverse phase PLC using methanol/water mixtures as an eluant.
Yield = 73.30 g (48%)

### EXAMPLE 19

### N,N'-Bis-{3,5-bis-[N-(2,3-dihydroxypropyl)carbamoyl]-2,4,6-triiodophenyl}-N,N'-dimethyl-3-(2-methoxyethyl)-3-azapentanediamide (ICJ 2892)

To a suspension of 138.5 g (0.16 mole) of the chlorine derivative described in the example 7A and 6.0 g (0.08 mole) of 2-methoxyethylamine in 420 ml of dioxane, 26.3 g (0.16 mole) of potassium iodide and 16.0 g (0.16 mole) of triethylamine are added. The reaction mixture is heated at 80 - 85°C for 24 hours, filtered and the solvent evaporated off at reduced pressure. The residue is dissolved in a water/methanol mixture and the protecting groups hydrolyzed using hydrochloric acid at pH 1-2 and 40°C for 3 hours. It is neutralized and the resulting solution evaporated to dryness. The residue is then purified by reverse phase PLC using methanol/water mixtures as an eluant.
Yield = 64.0 g (50%)

### EXAMPLE 20

### N,N'-Bis-{3,5-bis-[N-(2,3-dihydroxypropyl)carbamoyl]-2,4,6-triiodophenyl}-N,N'-dimethyl-3-methyl-3-azapentanediamide (ICJ 2992)

To a suspension of 175.1 g (0.2 mole) of the chlorine derivative described in the example 7A, and 3.1 g (0.1 mole) of methylamine in 525 ml of dioxane, 33.2 g (0.2 mole) of potassium iodide and 20.3 g (0.2 mole) of triethylamine are added. The reaction mixture is heated at 80 - 85°C for 24 hours, filtered and the solution evaporated to dryness at reduced pressure. The residue is dissolved in a water-methanol mixture and the protecting groups hydrolyzed using hydrochloric acid at pH 1-2 and 40°C for 3 hours. The residue is purified by reverse phase PLC using methanol-water mixtures as an eluant.
Yield = 82.0 g (53%)

## Claims

1. Non-ionic dimeric compounds derived from dicarbamoyl triiodoisophthalic acid of the general formula I 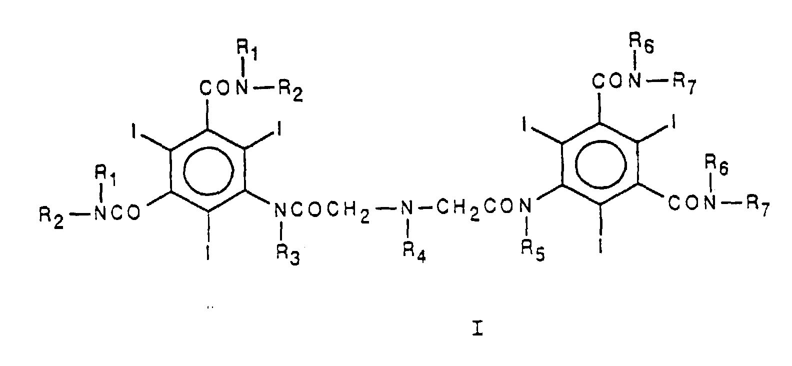 wherein
- R₁ and R₇, whether the same or diferent, are polyhydroxyalkyl radicals, linear or branched, from C₂ to C₄, with one to three OH groups;
- R₂ and R₆, whether the same or different, are hydrogen, methyl or 2-hydroxyethyl;
- R₃ and R₅, whether the same or different, are hydrogen or methyl;
- R₄ is hydrogen, methyl, methoxymethyl or a polyhydroxyalkyl radical, linear or branched, from C₂ to C₄, with one to three OH groups.

2. N,N'-Bis-{3,5-bis-[N-(2,3-dihydroxypropyl)carbamoyl]-2,4,6-triiodophenyl}-3-(2,3-dihydroxypropyl)-3-azapen-tanediamide.

3. N,N'-Bis-{3,5-bis-[N-(2,3-dihydroxypropyl)carbamoyl]-2,4,6-triiodophenyl}-3-(2-hydroxyethyl)-3-azapentanediamide.

4. N-{3,5-Bis-[N-(2,3-dihydroxypropyl)carbamoyl]-2,4,6-triiodophenyl}-N'-{3,5-bis-[N-(2-hydroxyethyl)carbamoyl]-2,4,6-triiodophenyl}-3-(2-hydroxyethyl)-3-azapentanediamide.

5. N,N'-Bis-{3,5-bis-[N-(2-hydroxyethyl)carbamoyl]-2,4,6-triiodophenyl}-3-(2-hydroxyethyl)-3-azapentanediamide.

6. N-{3,5-Bis-[N-(2,3-dihydroxypropyl)carbamoyl]-2,4,6-triiodophenyl}-N'-{3,5-bis-[N-(2-hydroxyethyl) carbamoyl]-2,4,6-triiodophenyl}-3-(2,3-dihydroxypropyl)-3-azapentanediamide.

7. N-{3,5-Bis-[N-(1-hydroxymethyl-2,3-dihydroxypropyl)carbamoyl]-2,4,6-triiodophenyl}-N'-{3,5-bis-[N-(2-hydroxyethyl)carbamoyl]-2,4,6-triiodophenyl}-3-(2-hydroxyethyl)-3-azapentanediamide.

8. N,N'-Bis-{3,5-bis-[N-(2,3-dihydroxypropyl)carbamoyl]-2,4,6-triiodophenyl}-N,N'-dimethyl-3-(2-hydroxyethyl)-3 -azapentanediamide.

9. N,N'-Bis-{3,5-bis-[N-(2,3-dihydroxypropyl)carbamoyl]-2,4,6-triiodophenyl}-N-methyl-3-(2-hydroxyethyl)-3-azapentanediamide.

10. N-{3,5-Bis-[N-(1-hydroxymethyl-2,3-dihydroxypropyl)carbamoyl]-2,4,6-triiodophenyl}-N-methyl-N'-{3,5-bis-[N-(2-hydroxyethyl)carbamoyl]-2,4,6-triiodophenyl}-3-(2-hydroxyethyl)-3-azapentanediamide.

11. N-{3,5-Bis-[N-(1-hydroxymethyl-2,3-dihydroxypropyl)carbamoyl]-2,4,6-triiodophenyl}-N'-methyl-N'-{3,5-bis-[N-(2-hydroxyethyl)carbamoyl]-2,4,6-triiodophenyl}-3-(2-hydroxyethyl)-3-azapentanediamide.

12. N-{3,5-Bis-[N-(1-hydroxymethyl-2,3-dihydroxypropyl)carbamoyl]-2,4,6-triiodophenyl}-N'-{3,5-bis-[N-(2-hydroxyethyl)carbamoyl]-2,4.6-triiodophenyl}-N,N'-dimethyl-3-(2-hydroxyethyl)-3-azapentanediamide.

13. N-{3,5-Bis-[N-(2,3-dihydroxypropyl)carbamoyl]-2,4,6-triiodophenyl}-N'-methyl-N'-{3,5-bis-[N-(2-hydroxyethyl)carbamoyl]-2,4,6-triiodophenyl}-3-(2-hydroxyethyl)-3-azapentanediamide.

14. N-{3,5-Bis-[N-(2,3-dihidroxypropyl)carbamoyl]-2,4,6-triiodophenyl}-N'-{3,5-bis-[N-(2'-hydroxyethyl)carbamoyl]-2,4,6-triiodophenyl}-N,N'-dimethyl-3-(2-hydroxyethyl)-3-azapentanediamide.

15. N,N'-Bis-{3,5-bis-[N-(2-hydroxyethyl)carbamoyl]-2,4,6-triiodophenyl}-N,N'-dimethyl-3-(2-hydroxyethyl)-3-azapentanediamide.

16. N-{3,5-Bis-[N-(2,3-dihydroxypropyl)carbamoyl]-2,4,6-triiodophenyl}-N-methyl-N'-{3,5-bis-[N-(2-hydroxyethyl)carbamoyl]-2,4,6-triiodophenyl}-3-(2-hydroxyethyl)-3-azapentanediamide.

17. N,N'-Bis-{3,5-bis-[N-(2-hydroxyethyl)carbamoyl]-2,4,6-triiodophenyl}-3-(1-hydroxymethyl-2,3-dihydroxypropyl)-3 -azapentanediamide.

18. N,N'-Bis-{3,5-bis-[N-(2,3-dihydroxypropyl)carbamoyl]-2,4,6-triiodophenyl}-3-(2-methoxyethyl)-3-azapentanediamide.

19. N,N'-Bis-{3,5-bis-[N-(2,3-dihydroxypropyl)carbamoyl]-2,4,6-triiodophenyl}-3-methyl-3-azapentanediamide.

20. N,N'-Bis-{3,5-bis-[N-(2,3-dihydroxypropyl)carbamoyl]-2,4,6-triiodophenyl}-N,N'-dimethyl-3-(2-methoxyethyl)-3 -azapentanediamide.

21. N,N'-Bis-{3,5-bis-[N-(2,3-dihydroxypropyl)carbamoyl]-2,4,6-triiodophenyl}-N,N'-dimethyl-3-methyl-3-azapentanediamide.

22. Galenical compositions which include some of the compounds listed in claims 1 - 19 in concentrations of between 100 - 400 mg I/ml and include pH stabilizers such as Tris or trometamol buffer (tris(hydroxymethyl)aminomethane) and their salts, phosphates, citrates and hydrogencarbonates as well as sterile apyrogenous water, being also possibly included heavy metal complexing agents, such as sodium and/or calcium salts of ethylenediaminetetraacetic acid and other chelating agents which are pharmaceutically acceptable.

23. Method for preparing compounds of the general formula I defined by claim 1, which comprises reacting a compound of the general formula II 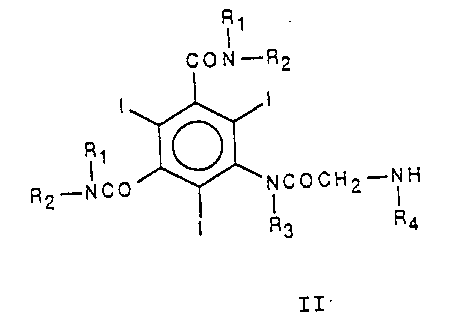 with a halogenated derivative of the formula III 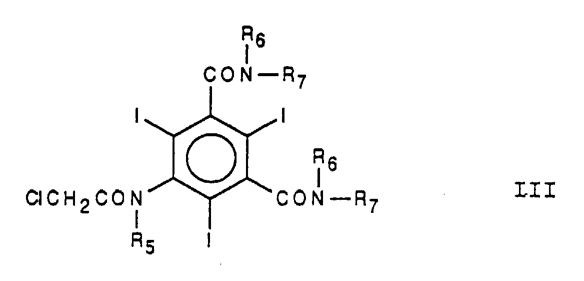 wherein R₁ to R₇ are the same as indicated for claim 1, in the presence of a basic catalyst, being protected the OH groups during the reaction as acetates or dioxepines and as dioxolanes when the OH groups are vicinal. These protecting groups are later removed by hydrolysis, thus producing the compounds of the general formula I.

## Patentansprüche

1. Nicht-ionische dimere Verbindungen, die von Dicarbamoyltrijodisophthalsäure nach der allgemeinen Formel I 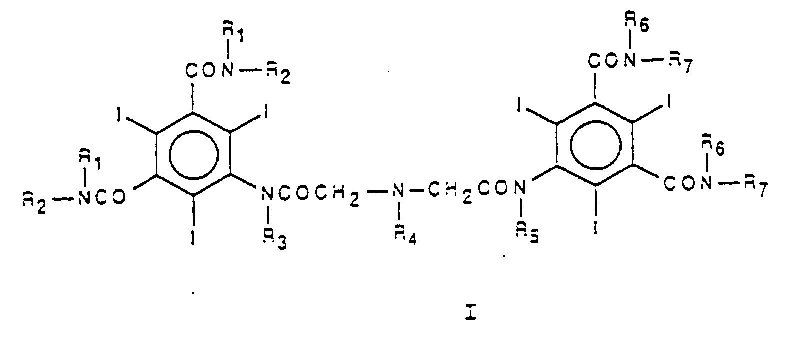 abgeleitet sind, wobei
- R₁ und R₇ gleich oder unterschiedlich sein können und geradkettige oder verzweigte C₂ bis C₄ Polyhydroxyalkylreste mit einer bis drei OH Gruppen sind,
- R₂ und R₆ gleich oder unterschiedlich sein können und Wasserstoff, Methyl oder 2-Hydroxyethyl sind,
- R₃ und R₅ gleich oder unterschiedlich sein können und Wasserstoff oder Methyl sind,
- R₄ Wasserstoff, Methyl, Methoxymethyl oder ein geradkettiger oder verzweigter C₂ bis C₄ Polyhydroxyalkylrest mit einer bis drei OH Gruppen ist.

2. N,N'-bis-{3,5-bis-[N-(2,3-dihydroxypropyl)carbamoyl]-2,4,6-trijodphenyl}-3-(2,3-dihydroxypropyl)-3-azapentandiamid.

3. N,N'-bis-{3,5-bis-[N-(2,3-dihydroxypropyl)carbamoyl]-2,4,6-trijodphenyl}-3-(2-hydroxyethyl)-3-azapentandiamid.

4. N-{3,5-bis-[N-(2,3-dihydroxypropyl)carbamoyl]-2,4,6-trijodphenyl}-N'-{3,5-bis-[N-(2-hydroxyethyl)carbamoyl]-2,4,6-trijodphenyl}-3-(2-hydroxyethyl)-3-azapentandiamid.

5. N,N'-bis-{3,5-bis-[N-(2-hydroxyethyl)carbamoyl]-2,4,6-trijodphenyl}-3-(2-hydroxyethyl)-3-azapentandiamid.

6. N-{3,5-bis-[N-(2,3-dihydroxypropyl)carbamoyl]-2,4,6-trijodphenyl}-N'-{3,5-bis-[N-(2-hydroxyethyl)carbamoyl]-2,4,6-trijodphenyl}-3-(2,3-dihydroxypropyl)-3-azapentandiamid.

7. N-{3,5-bis-[N-(1-hydroxymethyl-2,3-dihydroxypropyl)carbamoyl]-2,4,6-trijodphenyl}-N'-{3,5-bis-[N-(2-hydroxyethyl)carbamoyl]-2,4,6-trijodphenyl}-3-(2-hydroxyethyl)-3-azapentandiamid.

8. N,N'-bis-{3,5-bis-[N-(2,3-dihydroxypropyl)carbamoyl]-2,4,6-trijodphenyl}-N,N'-dimethyl-3-(2-hydroxyethyl)-3-azapentandiamid.

9. N,N'-bis-{3,5-bis-[N-(2,3-dihydroxypropyl)carbamoyl]-2,4,6-trijodphenyl}-N-methyl-3-(2-hydroxyethyl)-3-azapentandiamid.

10. N-{3,5-bis-[N-(1-hydroxymethyl-2,3-dihydroxypropyl)carbamoyl]-2,4,6-trijodphenyl}-N-methyl-N'-{3,5-bis-[N-(2-hydroxyethyl)carbamoyl]-2,4,6-trijodphenyl}-3-(2-hydroxyethyl)-3-azapentandiamid.

11. N-{3,5-bis-[N-(1-hydroxymethyl-2,3-dihydroxypropyl)carbamoyl]-2,4,6-trijodphenyl}-N'-methyl-N'-{3,5-bis-[N-(2-hydroxyethyl)carbamoyl]-2,4,6-trijodphenyl}-3-(2-hydroxyethyl)-3-azapentandiamid.

12. N-{3,5-bis-[N-(1-hydroxymethyl-2,3-dihydroxypropyl)carbamoyl]-2,4,6-trijodphenyl}-N'-(3,5-bis-[N-(2-hydroxyethyl)carbamoyl]-2,4,6-trijodphenyl}-N,N'-dimethyl-3-(2-hydroxyethyl)-3-azapentandiamid.

13. N-{3,5-bis-[N-(2,3-dihydroxypropyl)carbamoyl]-2,4,6-trijodphenyl}-N'-methyl-N'-{3,5-bis-[N-(2-hydroxyethyl)carbamoyl]-2,4,6-trijodphenyl}-3-(2-hydroxyethyl)-3-azapentandiamid.

14. N-{3,5-bis-[N-(2,3-dihydroxypropyl)carbamoyl]-2,4,6-trijodphenyl}-N'-{3,5-bis-[N-(2-hydroxyethyl)carbamoyl]-2,4,6-trijodphenyl}-N,N'-dimethyl-3-(2-hydroxyethyl)-3-azapentandiamid.

15. N,N'-bis-{3,5-bis-[N-(2-hydroxyethyl)carbamoyl]-2,4,6-trijodphenyl}-N,N'-dimethyl-3-(2-hydroxyethyl)-3-azapentandiamid.

16. N-{3,5-bis-[N-(2,3-dihydroxypropyl)carbamoyl]-2,4,6-trijodphenyl}-N-methyl-N'-{3,5-bis-[N-(2-hydroxyethyl)carbamoyl]-2,4,6-trijodphenyl}-3-(2-hydroxyethyl)-3-azapentandiamid.

17. N,N'-bis-{3,5-bis-[N-(2-hydroxyethyl)carbamoyl]-2,4,6-trijodphenyl}-3-(1-hydroxymethyl-2,3-dihydroxypropyl)-3-azapentandiamid.

18. N,N'-bis-{3,5-bis-[N-(2,3-dihydroxypropyl)carbamoyl]-2,4,6-trijodphenyl}-3-(2-methoxyethyl)-3-azapentandiamid.

19. N,N'-bis-{3,5-bis-[N-(2,3-dihydroxypropyl)carbamoyl]-2,4,6-trijodphenyl}-3-methyl-3-azapentandiamid.

20. N,N'-bis-{3,5-bis-[N-(2,3-dihydroxypropyl)carbamoyl]-2,4,6-trijodphenyl}-N,N'-dimethyl-3-(2-methoxyethyl)-3-azapentandiamid.

21. N,N'-bis-{3,5-bis-[N-(2,3-dihydroxypropyl)carbamoyl]-2,4,6-trijodphenyl}-N,N'-dimethyl-3-methyl-3-azapentandiamid.

22. Galenische Zusammensetzungen, die in den Ansprüchen 1 - 19 aufgeführte Verbindungen in Konzentrationen zwischen 100 - 400 mg I/ml und deren Salze, Phosphate, Citrate und Hydrogencarbonate enthalten und die pH-Stabilisatoren wie etwa Tris oder Trometamol Puffer (Tris(hydroxymethyl)aminomethan) enthalten sowie pyrogenfreies Wasser, wobei auch Schwermetalle komplexierende Mittel wie etwa Natrium- und/oder Calciumsalze von Ethylendiamintetraessigsäure und andere pharmazeutisch verträgliche Chelatbildner vorhanden sein können.

23. Verfahren zum Herstellen von Verbindungen der in Anspruch 1 definierten allgemeinen Formel I, welches umfaßt das Umsetzen einer Verbindung der allgemeinen Formel II 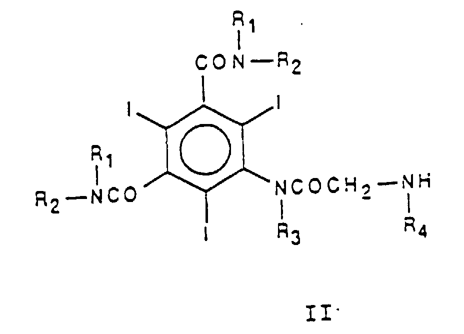 mit einem halogenierten Derivat der Formel III 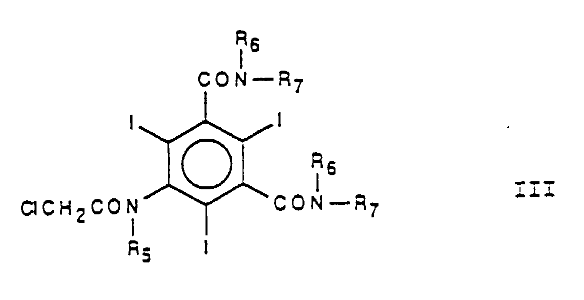 worin R₁ bis R₇ die in Anspruch 1 angegebenen Bedeutungen haben, in Gegenwart eines basischen Katalysators, wobei die OH-Gruppen während der Reaktion als Acetate oder Dioxepine, und wenn die OH-Gruppen vicinal sind, als Dioxolane geschützt werden, wobei diese Schutzgruppen später durch Hydrolyse entfernt werden, wodurch die Verbindungen der allgemeinen Formel I hergestellt werden.

## Revendications

1. Composés dimères non ioniques dérivés de l'acide dicarbamoyl triiodoisophtalique de formule générale I : 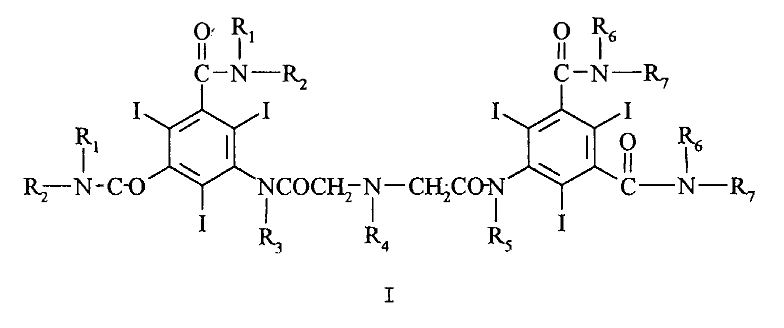 dans laquelle :
R₁ et R₇ sont identiques ou différents, et représentent des radicaux polyhydroxyalkyle en C₂-C₄ linéaires ou ramifiés, avec un à trois groupes OH;
R₂ et R₆ sont identiques ou différents, et représentent l'hydrogène, un radical méthyle, ou 2-hydroxyéthyle;
R₃ et R₅ sont identiques ou différents, et représentent l'hydrogène ou un radical méthyle;
R₄ représente l'hydrogène ou un radical méthyle, méthoxyméthyle ou polyhydroxyalkyle en C₂-C₄ linéaire ou ramifié, avec un à trois groupes OH.

2. N,N'-Bis-{3,5-bis-[N-(2,3-dihydroxypropyl)carbamoyl]-2,4,6-triiodophényl}-3-(2,3-dihydroxypropyl)-3-azapentanediamide.

3. N,N'-Bis-{3,5-bis-[N-(2,3-dihydroxypropyl)carbamoyl]-2,4,6-triiodophényl}-3-(2-hydroxyéthyl)-3-azapentanediamide.

4. N-{3,5-Bis-[N-(2,3-dihydroxypropyl)carbamoyl]-2,4,6-triiodophényl}-N'-{3,5-bis-[N-(2-hydroxyéthyl)carbamoyl]-2,4,6-triiodophényl}-3-(2-hydroxyéthyl)-3-azapentanediamide.

5. N,N'-Bis-{3,5-bis-[N-(2-hydroxyéthyl)carbamoyl]-2,4,6-triiodophényl}-3-(2-hydroxyéthyl)-3-azapentanediamide.

6. N-{3,5-Bis-[N-(2,3-dihydroxypropyl)carbamoyl]-2,4,6-triiodophényl}-N'-{3,5-bis-[N-(2-hydroxyéthyl)carbamoyl]-2,4,6-triiodophényl}-3-(2,3-dihydroxypropyl)-3-azapentanediamide.

7. N-{3,5-Bis-[N-(1-hydroxyméthyl-2,3-dihydroxypropyl)carbamoyl]-2,4,6-triiodophényl}-N'-{3,5-bis-[N-(2-hydroxyéthyl)carbamoyl]-2,4,6-triiodophényl}-3-(2-hydroxyéthyl)-3-azapentanediamide.

8. N,N'-Bis-{3,5-bis-[N-(2,3-dihydroxypropyl)carbamoyl]-2,4,6-triiodophényl}-N,N'-diméthyl-3-(2-hydroxyéthyl)-3-azapentanediamide.

9. N,N'-Bis-{3,5-bis-[N-(2,3-dihydroxypropyl)carbamoyl]-2,4,6-triiodophényl}-N'-méthyl-3-(2-hydroxyéthyl)-3-azapentanediamide.

10. N-{3,5-Bis-[N-(1-hydroxyméthyl-2,3-dihydroxypropyl)carbamoyl]-2,4,6-triiodophényl}-N-méthyl-N'-{3,5-bis-[N-(2-hydroxyéthyl)carbamoyl]-2,4,6-triiodophényl}-3-(2-hydroxyéthyl)-3-azapentanediamide.

11. N-{3,5-Bis-[N-(1-hydroxyméthyl-2,3-dihydroxypropyl)carbamoyl]-2,4,6-triiodophényl}-N'-méthyl-N'-{3,5-bis-[N-(2-hydroxyéthyl)carbamoyl]-2,4,6-triiodophényl}-3-(2-hydroxyéthyl)-3-azapentanediamide.

12. N-{3,5-Bis-[N-(1-hydroxyméthyl-2,3-dihydroxypropyl)carbamoyl]-2,4,6-triiodophényl}-N'-{3,5-bis-[N-(2-hydroxyéthyl)carbamoyl]-2,4,6-triiodophényl}-N,N'-diméthyl-3-(2-hydroxyéthyl)-3-azapentanediamide.

13. N-{3,5-Bis-[N-(2,3-dihydroxypropyl)carbamoyl]-2,4,6-triiodophényl}-N'-méthyl-N'-{3,5-bis-[N-(2-hydroxyéthyl)carbamoyl]-2,4,6-triiodophényl}-3-(2-hydroxyéthyl)-3-azapentanediamide.

14. N-{3,5-Bis-[N-(2,3-dihydroxypropyl)carbamoyl]-2,4,6-triiodophényl}-N'-{3,5-bis-[N-(2-hydroxyéthyl)carbamoyl]-2,4,6-triiodophényl}-N,N'-diméthyl-3-(2-hydroxyéthyl)-3-azapentanediamide.

15. N,N'-Bis-{3,5-bis-[N-(2-hydroxyéthyl)carbamoyl]-2,4,6-triiodophényl}-N,N'-diméthyl-3-(2-hydroxyéthyl)-3-azapentanediamide.

16. N-{3,5-Bis-[N-(2,3-dihydroxypropyl)carbamoyl]-2,4,6-triiodophényl}-N-méthyl-N'-{3,5-bis-[N-(2-hydroxyéthyl)carbamoyl]-2,4,6-triiodophényl}-3-(2-hydroxyéthyl)-3-azapentanediamide.

17. N,N'-Bis-{3,5-bis-[N-(2-hydroxyéthyl)carbamoyl]-2,4,6-triiodophényl}-3-(1-hydroxyméthyl-2,3-dihydroxypropyl)-3-azapentanediamide.

18. N,N'-Bis-{3,5-bis-[N-(2,3-dihydroxypropyl)carbamoyl]-2,4,6-triiodophényl}-3-(2-méthoxyéthyl)-3-azapentanediamide.

19. N,N'-Bis-{3,5-bis-[N-(2,3-dihydroxypropyl)carbamoyl]-2,4,6-triiodophényl}-3-méthyl-3-azapentanediamide.

20. N,N'-Bis-{3,5-bis-[N-(2,3-dihydroxypropyl)carbamoyl]-2,4,6-triiodophényl}-N,N'-diméthyl-3-(2-méthoxyéthyl)-3-azapentanediamide.

21. N,N'-Bis-{3,5-bis-[N-(2,3-dihydroxypropyl)carbamoyl]-2,4,6-triiodophényl}-N,N'-diméthyl-3-méthyl-3-azapentanediamide.

22. Compositions galéniques qui comprennent certains des composés listés dans les revendications 1 à 19 en des concentrations comprises entre 100 et 400 mg I/mL et qui comprennent des stabilisateurs de pH tels que le Tris ou le tampon trométanol (tris(hydroxyméthyl)aminométhane) et leurs sels, phosphates, citrates et hydrogénocarbonates ainsi que de l'eau stérile apyrogène, des agents complexants des métaux lourds étant aussi éventuellement inclus, tels que les sels de sodium et/ou de calcium de l'acide éthylène diaminetétraacétique et d'autres agents chélatants qui sont pharmaceutiquement acceptables.

23. Procédé de préparation des composés de formule générale I définie dans la revendication 1, qui comprend la réaction d'un composé de formule générale II : 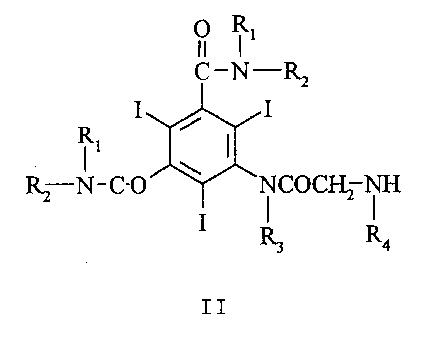 avec un dérivé halogéné de fomule III : 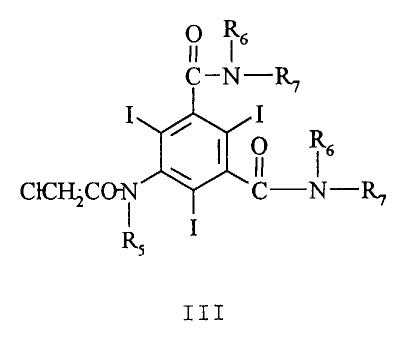 dans laquelle R₁ à R₇ sont tels que décrits dans la revendication 1, en présence d'un catalyseur basique, les groupes OH étant protégés durant la réaction en acétates ou dioxépines et en dioxolanes lorsque les groupes OH sont voisins. Ces groupes protecteurs sont plus tard éliminés par hydrolyse, donnant ainsi les composés de formule générale I.
